# EUROPEAN PATENT SPECIFICATION

(11) **EP 0 610 445 B1**
(45) Date of publication and mention of the grant of the patent: **05.01.2000**
(21) Application number: 93906309.5
(22) Date of filing: 28.09.1992
(51) Int. Cl.: A61K 38/00, A61K 9/127, A61K 47/24, A61K 47/26, G01N 33/92, A61L 27/00, A61L 29/00, A61L 31/00

(54) **USE OF COMPOSITIONS COMPRISING A BACTERICIDAL/PERMEABILITY INCREASING PROTEIN AND A LIPID CARRIER**
VERWENDUNG VON PROTEIN MIT ERHÖHTER BAKTERIENTÖTENDER/DURCHLASSIGKEITS-ERHÖHTEN WIRKUNG UND LIPID-TRÄGER ENTHALTENDER ZUSAMMENSETZUNG
UTILISATION DE COMPOSITIONS COMPRENANT UNE PROTEINE A ACTION BACTERICIDE/ACCROISSANT LA PERMEABILITE ET UN EXCIPIENT LIPIDIQUE

(30) Priority: 27.09.1991 US 766566
(43) Date of publication of application: 17.08.1994
(73) Proprietor: INCYTE PHARMACEUTICALS, INC., Palo Alto , CA 94304 (US)
(72) Inventor: MARRA, Marian, N., San Mateo, CA 94403 (US); SCOTT, Randal, W., Cupertino, CA 95014 (US); SNABLE, James L., Sunnyvale, CA 94086 (US); WILDE, Craig, G., Mountain View, CA 94040 (US)
(74) Representative: Henkel, Feiler, Hänzel
(86) International application number: PCT/US92/08234
(87) International publication number: WO 93/05797

(56) References cited:
- WO-A-87/04347
- WO-A-90/07950
- WO-A-90/09183
- WO-A-91/04019
- DE-A- 2 652 636
- US-A- 3 987 797
- US-A- 4 143 423
- US-A- 4 863 740
- US-A- 5 089 274

## Description

### Background of the Invention

Gram negative infections are a major cause of morbidity and mortality, especially in hospitalized and immunocompromised patients. [Duma, R.J., Am. J. of Med., 78 (Suppl. 6A): 154-164 (1985); and Kreger B.E., D.E. Craven and W.R. McCabe, Am. J. Med., 68: 344-355 (1980)]. Although available antibiotics are generally effective in containing the infection, they do nothing to neutralize the pathophysiological effects associated with lipopolysaccharide (LPS).

LPS, or endotoxin, is a major component of the outer membrane of gram negative bacteria and is released when the organisms are lysed [Ahenep, J.L. and K.A. Morgan, J. Infect. Dis., 150 (3): 380-388 (1984)].

LPS released during antibiotic therapy is a potent stimulator of the inflammatory response. Many detrimental effects of LPS in vivo result from soluble mediators released by inflammatory cells (Morrison D.C. and R.J. Ulevich, Am. J. Pathol., 93 (2): 527-617 (1978)]. LPS induces the release of mediators by host inflammatory cells which may ultimately result in disseminated intravascular coagulation (DIC), adult respiratory distress syndrome (ARDS), cardiac dysfunction, organ failure, liver failure (hepatobiliary dysfunction), brain failure (CNS dysfunction), renal failure, multi-organ failure and shock.

Soluble LPS causes decreased neutrophil chemotaxis, increased adhesiveness, elevated hexose monophosphate shunt activity and O₂ radical production, upregulation of surface receptors for complement, and release of granule proteins into the surrounding medium [Morrison and Ulevich (1978)].

Both specific and azurophil compartments degranulate in response to LPS (Bannatyne, R.M., N.M. Harnett, K.Y. Lee and W.D. Rigger, J. Infect. Dis., 156 (4): 469-474 (1977)). Azurophil proteins released in response to LPS may be both harmful and beneficial to the host. Neutrophil elastase causes degradation of protease inhibitors responsible for suppressing the coagulation cascade. This results in coagulopathies such as disseminated intravascular coagulation, a potentially lethal consequence of endotoxemia. Azurophil granules also contain bactericidal molecules such as myeloperoxidase and naturally occurring BPI Protein.

Endotoxemia is a condition associated with the presence of endotoxins, i.e. heat stable bacterial toxins, in the blood. Endotoxins elicit an inflammatory response that is beneficial in fighting the infection but can be damaging to the host if uncontrolled. Endotoxemia induces production of endotoxin-binding proteins from the liver and causes release of microbicidal proteins from leukocytes. Studies show that one of these leukocyte proteins, i.e. BPI Protein, previously known only for its bactericidal activity in vitro, inhibits the ability of endotoxin to stimulate neutrophils and monocytes in vitro and reduces death due to endotoxin or bacterial challenge when given in vitro.

Monocytes and neutrophilic granulocytes play a key role in host defense against bacterial infections and also participate in the pathology of endotoxemia. These cells ingest and kill microorganisms intracellularly and also respond to endotoxin in vivo and in vitro by releasing soluble proteins with microbicidal, proteolytic, opsonic, pyrogenic, complement-activating and tissue-damaging effects.

Tumor necrosis factor (TNF), a cytokine released by endotoxin-stimulated monocytes, mimics some of the toxic effects of endotoxin in vivo. Injecting animals with TNF causes fever, shock and alterations in glucose metabolism. TNF is also a potent stimulator of neutrophils. Other cytokines such as IL-1, IL-6, and IL-8 also mediate some of the pathophysiologic effects of LPS.

Despite improvements in antibiotic therapy, morbidity and mortality associated with endotoxemia remains high. Antibiotics alone are not effective in neutralizing the toxic effects of LPS. Therefore, the need arises for therapy with direct endotoxin-neutralizing activity.

Current methods for treatment of endotoxemia use antibiotics and supportive care. Most available adjunct therapies treat symptoms of endotoxic shock such as low blood pressure and fever but do not inactivate endotoxin. Other therapies inhibit inflammatory host responses to LPS. As indicated below, present therapies have major limitations due to toxicity, immunogenicity, or irreproducible efficacy between animal models and human trials.

Polymyxin B (PMB) is a basic polypeptide antibiotic which has been shown to bind to, and structurally disrupt, the most toxic and biologically active component of endotoxin, Lipid A. PMB has been shown to inhibit endotoxin activation of neutrophil granule release in vitro and is a potential treatment for gram negative infections in humans. However, because of its systemic toxicity, this drug has limited use except as a topical agent.

Combination therapy using antibiotics and high doses of methylprednisolone sodium succinate (MPSS) has been shown to prevent death in an experimental model of gram negative sepsis using dogs. Another study using MPSS with antibiotics in a multicenter, double blind, placebo-controlled, clinical study in 223 patients with clinical signs of systemic sepsis concluded that mortality was not significantly different between the treatment and placebo groups. Further, the investigators found that resolution of secondary infection within 14 days was significantly higher in the placebo group.

A relatively new approach to treatment of endotoxemia is passive immunization with endotoxin-neutralizing antibodies. Hyperimmune human immunoglobulin against E. coli J5 has been shown to reduce mortality in patients with gram negative bacteremia and shock by 50%. Other groups have shown promising results in animal models using mouse, chimeric, and human monoclonal antibodies. Although monoclonal antibodies have advantages over hyperimmune sera, e.g. more consistent drug potency and decreased transmission of human pathogens, there are still many problems associated with administering immunoglobulin to neutralize LPS. Host responses to the immunoglobulins themselves can result in hypersensitivity. Tissue damage following complement activation and deposition of immune complexes is another concern in the use of therapies involving anti-endotoxin antibodies in septic patients.

BPI Protein is a neutrophil granule protein first discovered in 1975 [Weiss, J., R.C. Franson, S. Becherdite, K. Schmeidler, and P. Elsbach J. Clin. Invest., 55:33 (1975)]. BPI Protein, a 57kD protein, was obtained in highly purified form from human neutrophils in 1978 and was shown to increase membrane permeability and have bactericidal activity against gram negative bacteria when assayed in phosphate buffered saline in vitro [Weiss, J., et al. J. Biol. Chem, 253(8): 2664-2672 (1978)]. Weiss et al. [J. Biol. Chem. 254(21): 11010-11014 (1979)], further showed that BPI Protein increased phospholipase A2 activity, suggesting a proinflammatory activity for BPI Protein in addition to its in vitro bactericidal activity.

Rabbit BPI Protein was purified in 1979 (Elsbach et al. J. Biol. Chem 254(21): 11000-11009) and shown to have bactericidal and permeability increasing properties identical to those of BPI Protein from humans, providing a further source of material for study. Both BPI Protein from rabbit and human were shown to be effective against a variety of gram negative bacteria in vitro, including K1-encapsulated E. coli [Weiss et al. Infection and Immunity 38(3): 1149-1153, (1982)].

A role for lipopolysaccharide in the in vitro bactericidal action of BPI Protein was proposed in 1984 by Weiss et al. [J. Immunol. 132(6): 3109-3115, (1984)]. These investigators demonstrated that BPI Protein bound to the outer membrane of gram negative bacteria, caused extracellular release of LPS, and selectively stimulated LPS biosynthesis.

In 1984, a protein with properties similar to those of BPI Protein was isolated from human neutrophils and designated cationic antimicrobial protein 57 (CAP 57) [Shafer, W.M., C.E. Martin and J.K. Spitznagel, Infect. Immun., 45:29 (1984)] This protein is identical to BPI Protein as determined by the N-terminal amino acid sequence, amino acid composition, molecular weight and source [Spitznagel et al., Blood 76:825-834, 1990]. Another group, Hovde and Gray, reported a bactericidal glycoprotein with virtually identical properties to BPI Protein in 1986 [Hovde and Gray, Infection and Immunity 54(1): 142-148 (1986)].

In 1985, Ooi et al. reported that BPI Protein retained its in vitro bactericidal activity after cleavage with neutrophil proteases, suggesting that fragments of the molecule retain activity [Ooi and Elsbach, Clinical Research 33(2):567A (1985)]. All of the in vitro bactericidal and permeability increasing activities of BPI Protein are present in the N-terminal 25kD fragment of the protein [Ooi, C.E., et al. J. Biol. Chem. 262: 14891 (1987)]

Evidence that BPI Protein binds to a structure associated with endotoxin on the outer membrane of bacteria is as follows: (1) increased sensitivity of rough strains of E. coli relative to smooth strains to the permeability increasing activities of BPI Protein [Weiss, J. et al. Infect. Immun. 51:594 (1986)]; (2) the Prm A mutation which results in altered endotoxin structure causes decreased binding of both polymyxin B (PMB) and BPI Protein [Farley, M. M. et al., Infect. Immun. 56:1536-1539 (1987) and Farley et al., Infect. Immun. 58:1589-1592 (1988)]; (3) PMB competes with BPI Protein for binding to S. typhimurium [Farley 1988]; and (4) BPI Protein shares amino acid sequence homology and immunocrossreactivity to another endotoxin-binding protein termed Lipopolysaccharide Binding Protein (LBP) [Tobias et al., J. Biol. Chem. 263(27): 13479-13481 (1988)].

LBP-LPS complexes bind to a cell surface receptor on monocytes (CD14) which results in increased synthesis and release of the inflammatory cytokine TNF (Schumann et al., Science 249:1429-1431]. Thus, LBP promotes the immunostimulatory activities of LPS. BPI Protein has exactly the opposite effect of LBP. BPI Protein binds LPS and inhibits neutrophil and monocyte activation [Marra et al., J. Immunol. 144:662-666 (1990); Marra and Scott, WO90/09183, published 23 August 1990; C.J. Fisher et al., Circulatory Shock 34: 120 (1991)].

A cDNA encoding BPI Protein was obtained and sequenced by Gray et al. [Gray et al., Clin. Res. 36:620A (1988) and Gray et al., J. Biol. Chem. 264(16):9505-9506 (1989)]. They reported that BPI Protein is a membrane protein which can be cleaved and released in soluble form as a 25kD fragment.

BPI Protein binding to gram negative bacteria was reported originally to disrupt LPS structure, alter microbial permeability to small hydrophobic molecules and cause cell death (Weiss, et al., 1978]. More recently, these same authors have demonstrated that such effects occur only in the absence of serum albumin. BPI Protein has no bactericidal activity when added to bacteria cultured in the presence of serum albumin, thus suggesting that BPI Protein does not kill bacteria in vivo where albumin is ubiquitous [Mannion et al., J. Clin. Invest. 85: 853-860 (1990) and Mannion et al., J. Clin. Invest. 86: 631-641]. Thus it has been previously understood in the art that the beneficial effects of BPI Protein are limited to in vitro bactericidal effects.

Further, BPI Protein is described by Gray et al. [J. Biol. Chem. 264(16):9505-9509 (1989)] as a membrane protein which must be cleaved to the 25kDa fragment to be released from the neutrophil granule membrane in soluble form.

BPI Protein is in fact a protein which binds LPS and inhibits the immunostimulatory and toxic activities of LPS in vitro and in vivo. Thus Bactericidal/Permeability Increasing Protein, which includes BPI Protein, has an important use in the therapeutic and prophylactic treatment of endotoxin-related disorders. Furthermore, Bactericidal/Permeability Increasing Protein has important uses in, inter alia, diagnostic procedures for detecting and quantitatively determining LPS in a sample, and in coating surfaces of devices with Bactericidal/Permeability Increasing Protein, where such surfaces are to come into contact with biological samples.

However, the prior art does not provide a satisfactory method for stably maintaining Bactericidal/Permeability Increasing Protein in a soluble, active form.

WO 90/09183 provides a method of inhibiting lipopolysaccharide (LPS)-mediated stimulation of cells by contacting the cells, in the presence of a cell-stimulating amount of lipopolysaccharide, with Bactericidal/Permeability Increasing Protein (BPI) in an amount effective to inhibit cell stimulation.

### Summary of the Invention

This invention provides a method of detecting lipopolysaccharide in a sample which comprises contacting the sample with a composition comprising a Bactericidal/Permeability Increasing Protein and a lipid carrier, wherein the Bactericidal/Permeability Increasing Protein is solubilized in the lipid carrier, under conditions such that a lipopolysaccharide-Bactericidal/Permeability Increasing Protein complex is formed, and detecting the lipopolysaccharide-Bactericidal/Permeability Increasing Protein complex, thereby detecting the lipopolysaccharide in the sample.

This invention further provides a method of quantitatively determining the amount of lipopolysaccharide in a sample which comprises contacting the sample with a composition comprislng a Bactericidal/Permeability Increasing Protein and a lipid carrier, wherein the Bactericidal/Permeability Increasing Protein is solubilized in the lipid carrier, under conditions such that a lipopolysaccharide-Bactericidal/Permeability Increasing Protein complex is formed, and quantitatively determining the amount of the lipopolysaccharide-Bactericidal/Permeability Increasing Protein complex, thereby quantitatively determining the amount of lipopolysaccharide in the sample.

Further, there is described a method of coating a surgical tool with a Bactericidal/Permeability Increasing Protein so that the Bactericidal/Permeability Increasing Protein is capable of forming a complex with unbound lipopolysaccharide, which method comprises contacting a pharmaceutical composition comprising a therapeutically effective amount of a Bactericidal/Permeability Increasing Protein and a pharmaceutically acceptable lipid carrier, wherein the Bactericidal/Permeability Increasing Protein is solubilized in the lipid carrier, with a surface of the tool, which surface is designed for contact with a biological sample, under conditions such that the Bactericidal/Permeability Increasing Protein therein will attach to the surface of the tool.

Further, there is described a method of coating an implantable, invasive device with a Bactericidal/Permeability Increasing Protein so that the Bactericidal/Permeability Increasing Protein is capable of forming a complex with unbound lipopolysaccharide, which method comprises contacting a pharmaceutical composition comprising a therapeutically effective amount of a Bactericidal/ Permeability Increasing Protein and a pharmaceutically acceptable lipid carrier, wherein the Bactericidal/Permeability Increasing Protein is solubilized in the lipid carrier, with a surface of the device, which surface is designed for contact with a biological sample, under conditions such that the Bactericidal/Permeability Increasing Protein therein will attach to the surface of the device.

Further, there is described a method of decontaminating a fluid containing lipopolysaccharide prior to administration of the fluid into a subject, which comprises contacting the fluid with a pharmaceutical composition comprising a therapeutically effective amount of a Bactericidal/ Permeability Increasing Protein and a pharmaceutically acceptable lipid carrier, wherein the Bactericidal/ Permeability Increasing Protein is solubilized in the lipid carrier, prior to administration, under conditions such that the lipopolysaccharide forms a complex with the Bactericidal/Permeability Increasing Protein therein, and separating the complex so formed from the fluid, thereby decontaminating the fluid.

### Brief Description of the Figures

### Figure 1

Inhibition of LPS Activity by rBPI Protein in the Chromogenic LAL Assay. E. coli 0111:B4 (0-1EU/ml) was pro-incubated for 1 hour at 37°C with 0-100 µg/ml BPI Protein, and then assayed for endotoxin activity in the chromogenic limulus amebocyte lysate (LAL) assay. Data shows assay values ± SD for endotoxin (ordinate) in endotoxin units (EU) per ml, vs the amount of endotoxin added into the incubation mixture (abscissa) in EU/ml.

### Figure 2

Schematic illustration of the pT7BPI protein plasmid construct.

### Figure 3

A nucleotide and amino acid sequence of BPI protein mutagenic primer 25 kD Pro 212 TGA which is a C-terminal truncation of BPI protein.

### Figure 4

A nucleotide and amino acid sequence of BPI protein mutagenic primer 38 kD Pro 337 TGA which is a C-terminal truncation of BPI protein.

### Figure 5

A nucleotide and amino acid sequence of BPI protein mutagenic primer: Preferred ATG 5' HindIII which is a C-terminal truncation of BPI protein.

### Figure 6

Protein Sequence of p337.

### Figure 7

Protein Sequence of p212.

### Figure 8

Schematic illustration of pAc373.

### Detailed Description of the Invention

Specifically, there is described a composition comprising a Bactericidal/Permeability Increasing Protein and a lipid carrier, wherein the Bactericidal/Permeability Increasing Protein is solubilized in the lipid carrier.

As used herein, Bactericidal/Permeability Increasing Protein means and includes 1) human BPI Protein and BPI Protein; 2) any biologically active polypeptide which has substantially the same amino acid sequence as, and the biological activity of, BPI Protein; 3) a biologically active fragment of BPI protein or polypeptide analogs of BPI Protein; and 4) biologically active variants of BPI Protein. In this respect biologically active means capable of inhibiting the pyrogenic response to LPS. This biological activity may be measured in the rabbit USP pyrogen assay.

As used herein, "human BPI Protein" or "BPI Protein" means a native or naturally-occurring 57 kD protein which binds to the outer membrane of susceptible gram negative bacteria.

As used herein, "biologically active fragment of BPI Protein" means a polypeptide of molecular weight less than 57kd, having the biological activity of, and an amino acid sequence present within, BPI Protein.

As used herein, "biologically active polypeptide analog of BPI Protein" means a polypeptide which has substantially the same amino acid sequence as, and the biological activity of, BPI Protein. Biologically active polypeptide analogs of BPI Protein include polypeptide, the sequence of which varies from the sequence of BPI Protein by a changed amino acid within the BPI Protein sequence, e.g. a mutation, or by the addition of one or more amino acids at the amino- or carboxy- terminus, or both, of the BPI Protein sequence.

As used herein, "biologically active variant of BPI Protein" means a polypeptide that (1) includes a portion of the amino acid sequence which is present within BPI Protein and an amino acid sequence which is not present within BPI Protein, and (2) has substantially the same biological activity, i.e. endotoxin-neutralizing activity, as BPI Protein. A biologically active variant of BPI Protein may be a recombinant BPI Protein.

As used herein, a "recombinant BPI Protein" means a polypeptide produced by genetic engineering methods. Thus, each of BPI Protein, biologically active polypeptide fragments of BPI Protein, biologically active polypeptide analogs of BPI Protein, and biologically active variants of BPI Protein may be recombinant. However, in the context of this application, BPI Protein is not the same as recombinant BPI Protein, the latter differing in some molecular characteristic from the native or naturally occurring polypeptide, e.g. in glycosylation pattern.

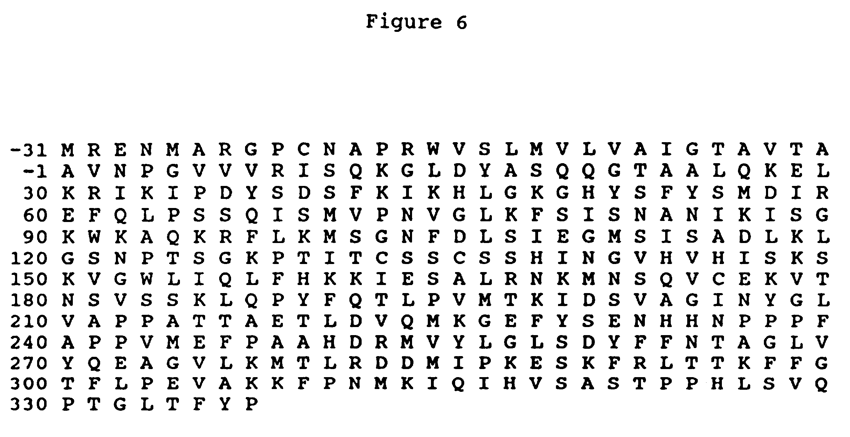

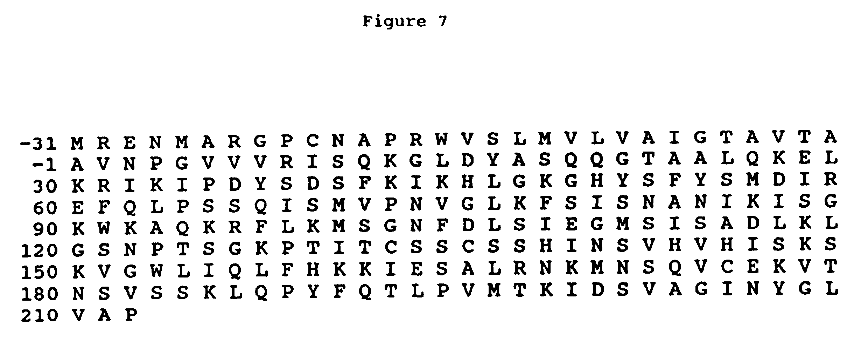

In addition, Bactericidal/Permeability Increasing Protein means and includes protein variants which (1) specifically bind to a lipid A domain of LPS, (2) compete with BPI Protein for binding to the lipid A binding site, (3) inhibit LPS-mediated stimulation of cells, and (4) do not exhibit microbiocidal activity. An example of a Bactericidal/ Permeability Increasing Protein variant is the protein variant shown in Figure 6. Another example of a Bactericidal/Permeability Increasing Protein variant is the protein variant shown in Figure 7.

As used herein, a "lipid carrier" is a liquid or gel comprising any fat-soluble substance capable of inhibiting the precipitation of hydrophobic proteins. Lipid carriers may also contain a buffered solution, salt (e.g. NaCl) and a protein carrier (e.g. serum albumin). Furthermore, lipid carriers may be in the form of solutions or gels. Such lipid carriers are formulated by methods well known to those skilled in the art.

As used herein, protein is "solubilized" in the lipid carrier when the surface area of a monomer or multimer of the protein is in contact with the lipid carrier. Protein which precipitates from solution is not solubilized. Solubility may be determined by absorbance at 280nm, whereby solubilized protein absorbs light at 280nm and precipitated protein does not.

In one embodiment, the lipid carrier comprises a liposome. As used herein, a "liposome" is any membrane bound vesicle which contains a desired substance, such as Bactericidal/Permeability Increasing Protein. Liposomes may also include cholesterol as a component.

In another embodiment, the lipid carrier comprises a non-ionic detergent. As used herein, a non-ionic detergent is any non-ionic lipid-soluble compound capable of suspending an otherwise insoluble substance in a lipid soluble environment. Examples of nonionic detergents include, but are not limited to, polysorbate 80 (also known as Tween 80 or polyoxyethylenesorbitan monooleate), Brij, polysorbate 80, and Triton (for example Triton WR-1339 and Triton A-20).

In another embodiment, the lipid carrier comprises a phospholipid. The phospholipid may be any naturally occurring mammalian phospholipid, such as phosphatidyl inositol or phosphatidyl choline.

There is also described a method of forming said composition, which comprises contacting a Bactericidal/Permeability Increasing Protein with & lipid carrier under conditions such that the Bactericidal/Permeability Increasing Protein is solubilized. Conditions for solubilizing a protein in a lipid carrier are well known to those skilled in the art.

There is further described a pharmaceutical composition comprising a therapeutically effective amount of a Bactericidal/Permeability Increasing Protein and a pharmaceutically acceptable lipid carrier, wherein the Bactericidal/Permeability Increasing Protein is solubilized in the lipid carrier.

As used herein, a "therapeutically effective amount of a Bactericidal/Permeability Increasing Protein" means an amount which, when administered to a subject, is capable of producing a desired result, i.e. treating or preventing endotoxemia or disorders related thereto. Methods of determining a therapeutically effective amount of a Bactericidal/Permeability Increasing Protein are well known to those of skill in the art.

Specifically, a therapeutically effective amount of a Bactericidal/Permeability Increasing Protein is an amount sufficient to achieve a concentration in a subject of between 0.1mg/kg and 100mg/kg. In the preferred embodiment, a therapeutically effective amount of a Bactericidal/Permeability Increasing Protein is an amount sufficient to achieve a concentration in a subject of between 1mg/kg and 10mg/kg.

For example, 350mg of Bactericidal/Permeability Increasing Protein is a therapeutically effective amount of Bactericidal/Permeability Increasing Protein to achieve a concentration of 5mg/kg in a 70kg subject.

As used herein, a "pharmaceutically acceptable lipid carrier" means a lipid carrier suitable for use as a medicinal product either for internal or external use. Such carriers are well known to those skilled in the art.

In one embodiment, the pharmaceutically acceptable lipid carrier comprises a liposome. In another embodiment of this invention, the pharmaceutically acceptable lipid carrier comprises a non-ionic detergent.

There is further described a method of forming said pharmaceutical composition which comprises contacting a pharmaceutically effective amount of a Bactericidal/Permeability Increasing Protein with a pharmaceutically acceptable lipid carrier under conditions such that the Bactericidal/Permeability Increasing Protein is solubilized. Conditions for solubilizing a protein in a lipid carrier are well known to those skilled in the art.

This invention provides a method of detecting lipopolysaccharide in a sample which comprises contacting the sample with the composition as mentioned above under conditions such that a lipopolysaccharide-Bactericidal/Permeability Increasing Protein complex is formed, and detecting the lipopolysaccharide-Bactericidal/Permeability Increasing Protein complex, thereby detecting the lipopolysaccharide in the sample.

Methods of detecting protein complexes are well known to those skilled in the art, and include, by way of example, ELISA and radioimmunoassay techniques.

This invention further provides a method of quantitatively determining the amount of lipopolysaccharide in a sample which comprises contacting the sample with the composition as mentioned above under conditions such that a lipopolysaccharide-Bactericidal/Permeability Increasing Protein complex is formed, and quantitatively determining the amount of the lipopolysaccharide-Bactericidal/Permeability Increasing Protein complex, thereby quantitatively determining the amount of lipopolysaccharide in the sample.

Methods of quantitatively determining protein complexes are well known to those skilled in the art, and include, by way of example, ELISA and radioimmunoassay techniques.

There is further described a method of coating a surgical tool with a Bactericidal/Permeability Increasing Protein so that the Bactericidal/Permeability Increasing Protein, is capable of forming a complex with unbound lipopolysaccharide, which method comprises contacting the pharmaceutical composition as mentioned above with a surface of the tool, which surface is designed for contact with a biological sample, under conditions such that the Bactericidal/Permeability Increasing Protein therein will attach to the' surface of the tool

Surgical tools include, by way of example, cutting instruments, forceps, suction apparati and catheters. Conditions under which a protein will attach to the surface of a tool are well known to those skilled in the art.

There is further described a method of coating an implantable, invasive device with a Bactericidal/Permeability Increasing Protein so that the Bactericidal/Permeability Increasing Protein is capable of forming a complex with unbound lipopolysaccharide, which method comprises contacting the pharmaceutical composition as mentioned above with a surface of the device, which surface is designed for contact with a biological sample, under conditions such that the Bactericidal/Permeability Increasing Protein therein will attach to the surface of the device.

Implantable, invasive devices include, by way of example, artificial joints, protective plates, and screws. Conditions under which a protein will attach to the surface of an implantable, invasive device are well known to those skilled in the art. In the preferred embodiment, a biological sample is an internal compartment of a human.

There is further described a method of decontaminating a fluid containing iipopolysaccharide prior to administration of the fluid into a subject, which comprises contacting the fluid with the pharmaceutical composition as mentioned above to administration, under conditions such that the lipopolysaccharide forms a complex with the Bactericidal/Permeability Increasing Protein therein, and separating the complex so formed from the fluid, thereby decontaminating the fluid.

As used herein, "decontaminating" a fluid containing lipopolysaccharide means removing the lipopolysaccharide therefrom.

As used herein, administration may be performed intravenously, intraperitoneally, or by any other suitable method of administration well known to those skilled in the art.

Conditions under which the lipopolysaccharide forms a complex with the Bactericidal/Permeability Increasing Protein are well known to those of skill in the art. Furthermore, separating the lipopolysaccharide-Bactericidal/Permeability Increasing Protein complex from the fluid may be accomplished by any suitable means known to those skilled in the art.

This invention will be better understood by reference to the Experimental Details which follow, but those skilled in the art will readily appreciate that the specific experiments detailed are only illustrative of the invention as described more fully in the claims which follow thereafter.

### Experimental Details

### Example 1:

Precipitation of recombinant BPI Protein (rBPI) by mechanical agitation. Sample of rBPI in 50mM Tris, 1M NaCl, pH 7.4 contained in half-full test tubes (generating a significant air-liquid interface) will precipitate within 30 minutes if agitated on a rocker platform (Labquake Shaker, Cat. No. 400-110 Lab Industries, Berkeley, CA). Similar efficiency of precipitation is observed upon vortexing of rBPI solution for 5 minutes. Typically, yields for either method of precipitation are approximately 97% precipitated.

The effect of different agents on rBPI solubility. Recombinant BPI (1.6 milligrams per milliliter) in 50mM Tris 1M NaCl pH 7.4 was diluted 10 fold into the following solutions and agitated overnight at 4°C using a rocker platform. Each solution was then observed for visible precipitation (Table I).

**TABLE I**

| Solution present (visual) | precipitate |
|---|---|
| 50mM Tris pH 7.4 100mM NaCl | X |
| 50mM Tris pH 7.4 1M NaCl | X |
| 50mM Tris pH 7.4 10mM DTT | X |
| 50mM Tris pH 7.4 10mM Ascorbic Acid | X |
| 50mM Tris pH 7.4 (under Argon) | X |
| 40mM Citrate pH 4 | X |
| 40mM Citrate pH 5 | X |
| 40mM Citrate pH 6 | X |
| 40mM Citrate pH 7 | X |
| 50mM Glycine pH 3 | - |
| 0111.B4 LPS 200µg/ml (pre-incubated 30' @ 38° | - |
| Bovine Serum Albumin 100µg/ml | X |
| Bovine Serum Albumin 1mg/ml | X |
| 100mM Arginine pH 8 | X |
| 1M Arginine pH 8 | X |
| 0.05% Triton X-114 | - |

| | |
|---|---|
| x = precipitate observed | |
| - = no precipitate observed | |

### Example 2

Effect of Various Lipid or Detergent Carriers on BPI Protein Solubility. The following reagents were added to rBPI (0.16 mg/ml final concentration) and the solution was agitated on a rocker platform for 30 minutes. Solubility was determined by absorbance at 280nm before and after agitation (Table 2).

**TABLE 2**

| Condition | % soluble |
|---|---|
| 0.5mM Triton X-114 | 90 |
| 0.1mM Triton X-114 | 59 |
| 0.003% Tween 80* | 3 |
| 0.0007% Tween 80 | 3 |
| 50mM Octyl Glucoside | 2 |
| 25mM Octyl Glucoside | 29 |
| 12.5mM Octyl Glucoside | 81 |
| 6.25mM Octyl Glucoside | 80 |
| 3.12mM Octyl Glucoside | 83 |
| 1.56mM Octyl Glucoside | 64 |
| 5% polyethylene glycol 6000 | 10 |
| 1% polyethylene glycol 6000 | 2 |
| 0.2% polyethylene glycol 6000 | 2 |
| 20% ethylene glycol | 9 |
| 100mM ammonium sulfate | 3 |
| 20% glycerol | 9 |
| 0.2% Brij 35* | 101 |
| 0.005% Brij 35 | 97 |
| 0.05% mixed alkyltrimethylammonium bromides | 105 |
| 1M Glucose | 0 |
| 1M Mannose | 0 |
| 1M Galactose | 5 |

| | |
|---|---|
| * Tween 80=polyoxyethylenesorbitan monooleate Brij 35 = Polyethylene glycol alkyl ether | |

### Example 3

Effect of Tween 80 (polyoxyethylenesorbitan monooleate) on BPI Protein solubility. Additional stability studies using the following concentrations of Tween 80 were performed as described in Example 2 (Table 3). Quantitation was by enzyme linked immunosorbent assay.

**TABLE 3**

| Condition | % soluble |
|---|---|
| 1.0% Tween 80 | 187 |
| 0.5% Tween 80 | 137 |
| 0.25% Tween 80 | 81 |
| 0.125% Tween 80 | 30 |
| 0.063% Tween 80 | 8 |
| 0.0031% Tween 80 | 1 |

### Example 4

BPI Protein in a lipid carrier, i.e. Tween 80, effectively protects mice from endotoxin lethality (Table 4).

**TABLE 4**

| Protection from endotoxin lethality by BPI Protein in CD-1 Mice (LPS-0111:B4 at 25 mg/kg IV) (Survivors/Total at 24 Hours) | |
|---|---|
| Dose of BPI (IV) | BPI in Formulation #1 |
| Saline control | 0/5 |
| 5 mg/kg | 5/5 |
| 10 mg/kg | 5/5 |
| Formulation #1: 20 mM Citrate, 150mM NaCl, 0.2% Tween 80, pH 6.0. | |

### Example 5

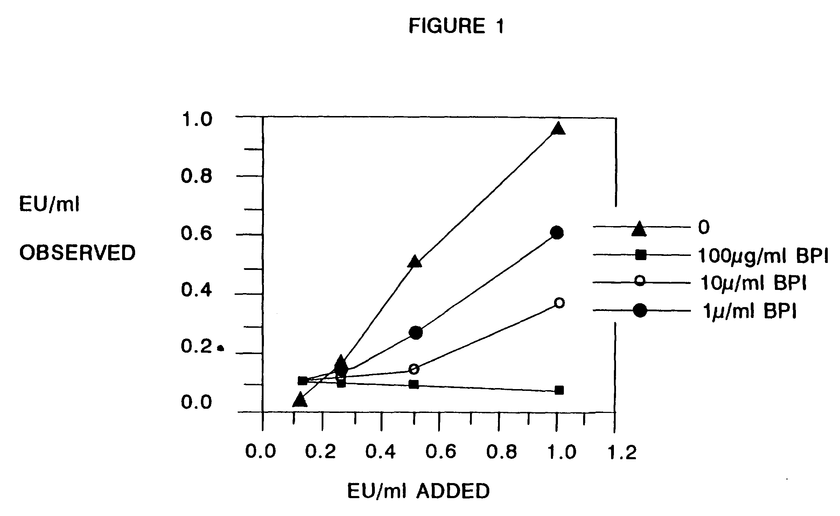

Inhibition of LPS Activity by rBPI in the Chromogenic LAL Assay. E. coli 0111:B4 (0-1EU/ml) was pre-incubated for 1 hour at 37°C with 0-100 µg/ml BPI Protein, and then assayed for endotoxin activity in the chromogenic limulus amebocyte lysate (LAL) essay. Data (Figure 1) shows assay values ± SD for endotoxin (ordinate) in endotoxin units (EU) per ml, vs the amount of endotoxin added into the incubation mixture (abscissa) in EU/ml.

### Example 6

Inhibition of LPS-Induced TNF Secretion by BPI Protein. Human peripheral blood mononuclear cells were isolated on Ficoll-Hypague gradients, washed 2X in pyrogen-free HBSS, and resuspended at 5 X 10⁶/ml in RPMI 1640 medium without serum. Two hundred microliters of this cell suspension was incubated in each well of flat-bottom 96-well tissue culture dishes for 2 hours at 37°C. Nonadherent cells were recombed by washing twice with warm RPMI 1640. Cells were cultured in RPMI 1640 + 10% autologous heat-inactivated serum. Adherent mononuclear cells were stimulated with E. coli 0111:B4 LPS which had been preincubated for 30 minutes at 37°C with buffer, BPI Protein or polymyxin B (7900 U/ml). Supernatants were harvested four hours after LPS mixtures were added. Secretion of TNFα was quantified by ELISA. Results are shown in Table 5.

**TABLE 5**

| Inhibition of LPS-Induced TNF Secretion by BPI Protein TNF pg/ml | | | | |
|---|---|---|---|---|
| LPS ng/ml | Buffer Control | rBPI ng/ml | | |
| | | 1000 | 100 | 10 |
| 1000 | 1728 ± 416 | 562 ± 47 | 1694 ± 134 | 2052 ± 324 |
| 100 | 1300 ± 199 | 66 ± 7 | 407 ± 99 | 1479 ± 232 |
| 10 | 826 ± 216 | 0 ± 16 | 0 ± 16 | 0 ± 56 |
| 1 | 232 ± 100 | 0 ± 16 | 0 ± 16 | 0 ± 10 |

### Example 7

### BPI protein Purification

A. Reagents
   Lipopolysaccharide from E. Coli 0111:B4, S. typhimurium wild type, glycolipid from S. typhimurium RE mutant, LPS from P. aeruginosa were purchased from RIBI Immmunochem Research, Inc., Hamilton, MT; Hank's Balanced Salt Solution without calcium, magnesium and phenol red (HBSS) from Hazelton Research Products, Denver, PA; Ficoll-Pague, Percoll and Macrodex from Pharmacia Inc., Piscataway, NJ; TNF and anti-TNF from Endogen, Boston MA.
B. Azurophil Granule Isolation and Extraction
   Granulocytes were isolated from buffy coats obtained from local blood banks. Buffy coats were diluted 3-4X in HBSS and granulocytes were separated from mononuclear cells by centrifugation through 64% Percoll. The pellet was subjected to diisopropylfluorophosphate (DFP), washed, and resuspended in ice cold lysis buffer (10 mM PIPES, pH 6.8, 100 mM KCL, 3mM NaCl, 3.5 mM MgCl₂) and disrupted by nitrogen cavitation (Parr Instrument Co., Moline, IL). Azurophil granules were isolated on discontinuous Percoll gradients. Such methods are well known to those skilled in the art. The azurophil granules were collected and Percoll was removed by centrifugation at 180,000 X G for 2 hours. The granules were lysed by 4 cycles of freeze-thaw followed by 1 minute of sonication. The lysed granules were extracted in an equal volume of 100 mM glycine, pH 2 by vortexing intermittently for 1 hour at room temperature. The acid extract was clarified by centrifugation at 30,000 X G for 20 minutes and at 200,000 X G for 30 minutes.
C. Neutrophil Isolation
   Venous blood was drawn from healthy volunteer donors into acid citrate dextrose anticoagulant and immediately placed on ice. Five parts of blood were mixed with 1 part of cold Macrodex, and allowed to settle for 1.5 - 2 hours at 4°C. Leukocyte-rich plasma was washed 1X in cold HBSS, then resuspended in HBSS and layered over Ficell-Paque. If significant erythrocyte contamination was present, the granulocyte pellet was subjected to hypotonic lysis. The cells were washed 2X in HBSS and resuspended in HBSS + 2% autologous plasma to give a final granulocyte concentration of 1 X 10⁶/ml in the incubation mixture
D. BPI Protein Purification
   Approximately 2 mg of crude azurophil granule extract was separated by size on a Biosil (TSK-250) (7.8 mm x 600 mm) high performance size exclusion column using 50 mM glycine and 100 mM NaCl buffer, pH 2.0, under isocratic conditions of a flow rate of 1 ml/min. Column fractions with the greatest LPS inhibitory activity contained a large proportion of the 57 KD species. These TSK fractions were pooled and run over an Aquapore weak cation exchange (WCX) column (2.1 mm X 30 mm) using 50 mM citrate, pH 5.5, and eluted in a gradient of 0-75%, of 50 mM citrate and 1 M NaCl (Buffer B) in 25 min, then 75-100% Buffer B in 5 min with a flow rate of 200 ml/min. Material of 57 KD was recovered from cation exchange and appeared as a single band on SDS page. In some experiments BPI protein was further purified by reverse phase HPLC on a Vydac C4 column loaded for 12 min in 0.1% CH₃CN plus 0.1% TFA, in 30 min with a flow rate of 200 ml/min (Rainin Instruments, Emeryville, CA).

### Example 8

### Expression of BPI Protein and BPI Protein Truncated Forms

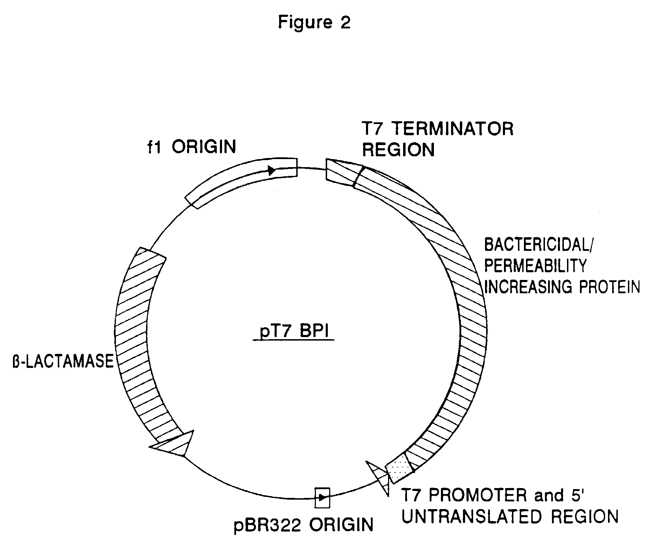

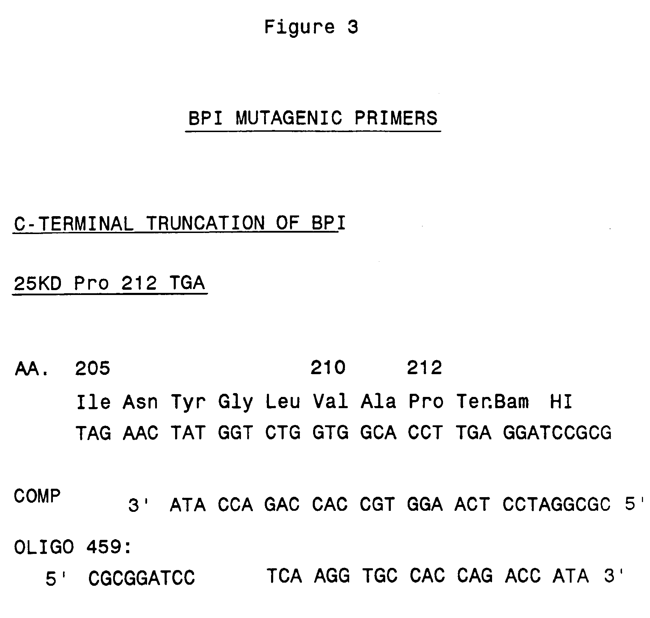

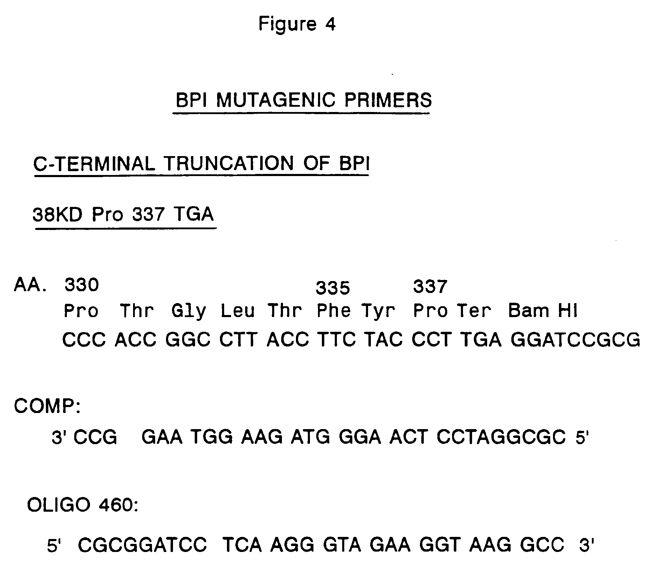

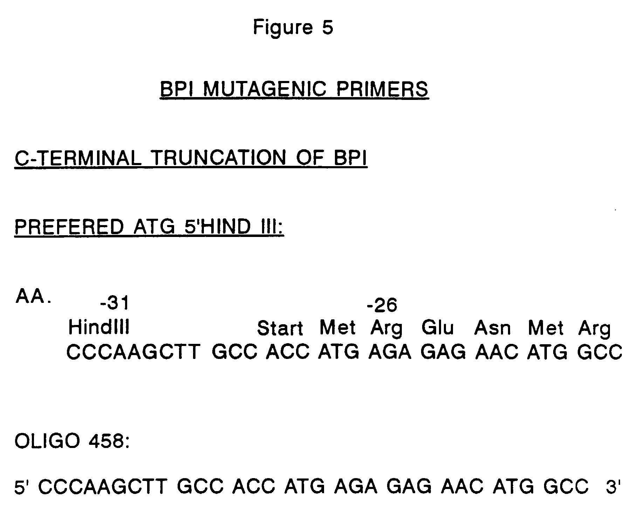

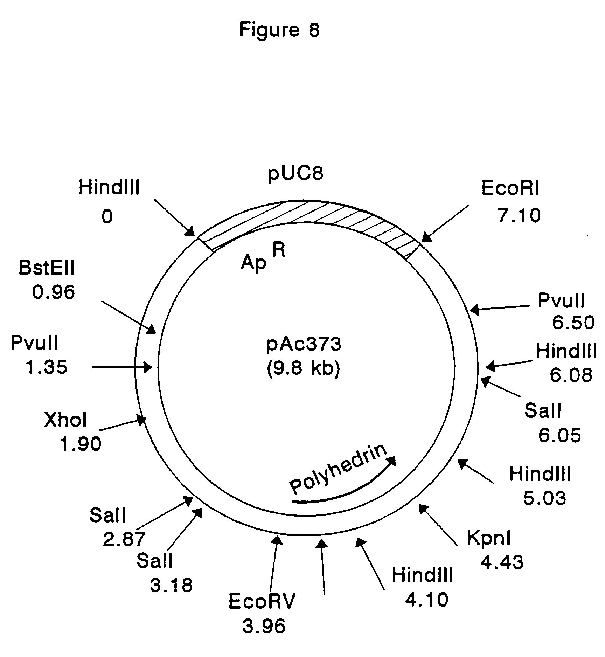

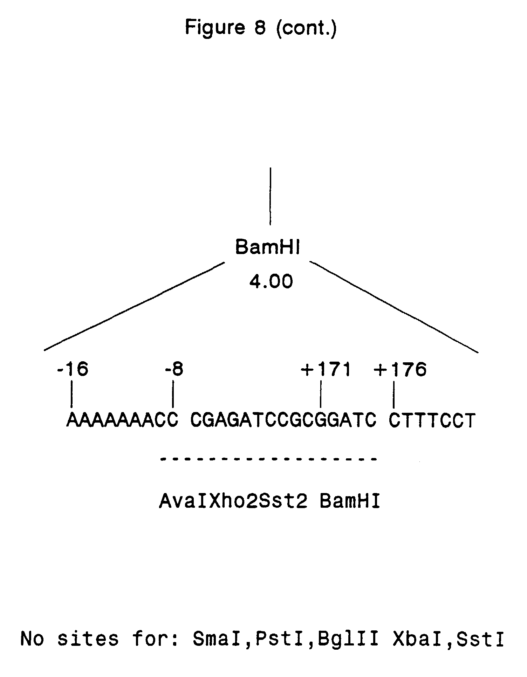

A. Genetically Engineered Mammalian Cells Express BPI Protein
   In order to produce BPI protein and/or BPI protein variants in mammalian cells, the cDNA sequences must be inserted into a suitable plasmid vector. A suitable vector for such an application is pSV-1, which contains the origin of replication and early and late promoters of SV40, followed by multiple insert cloning sites, followed by the termination sequences from the hepatitis B surface antigen gene (Figure 2). Also contained within the plasmid are an origin of bacterial DNA replication, and the genes encoding ampicillin resistance and dihydrofolate reductase. Similar vectors have been used to express other foreign genes (McGrogan, et.al. Biotechnology 6, 172-177). Vector DNA was prepared for acceptance of BPI protein cDNA sequences by digestion with HindIII and BamHI, and dephosphorylation with alkaline phosphatase.
   Several BPI protein cDNA-containing inserts were prepared for insertion into pSV-1. First, an insert encoding full-length BPI protein was prepared by digestion of the parent plasmid with EcoRI, yielding two DNA fragments containing portions of the BPI protein coding sequence. These two fragments were ligated together into prepared SV-1, and the recombinant clones obtained were screened by restriction enzyme digestion for the presence of the two inserts in the proper orientation. Two cDNAs encoding truncated forms of BPI protein were generated from parent BPI protein insert DNA. The amplifying oligos were designed to replace codons 212 (oligo 459) (Figure 3) and 337 (oligo 460) (Figure 4) with stop codons, in addition to a BamHI cloning site. At the 5'-end of both constructs, oligo 458 was used in the amplifications to create a HindIII site immediately upstream of the translational start codon ATG (Figure 5). Thus, three BPI-encoding inserts were created, each encoding 55 kD, 38 kD, and 25 kD forms of recombinant BPI Protein, and each was ligated separately into prepared vector DNA.
   Each of the three constructs was verified by restriction digest analysis, and then prepared in amounts sufficient for transfection into CHO cell line DUXB11 cells. Transfection was performed using lipofectin, and the resulting transformed cells were selected in the presence of increasing amounts of methotrexate using standard protocols. Supernatants from either transfected pools or clones derived from the pools were assayed for the presence of endotoxin-binding activity by ELISA.
B. Genetically Engineered Insect Cells Express BPI Protein. Construction of plasmid expression vector: In order to produce BPI Protein and/or BPI protein variants in insect cells, the cDNA sequence must first be inserted into a suitable plasmid expression vector, such as pAC373 (see Figure 8). Appropriate restriction sites for this insertion can be created by standard site-directed mutagenesis procedures. The essential properties of a suitable expression vector include a transcriptional promoter such as the polyhedron gene promoter of pAC373, and flanking homologous sequences to direct recombination into the baculovirus genome. A polyadenylation signal, such as the one form the polyhedron gene present in this plasmid vector, may or may not be necessary for expression of the recombinant gene. A marker gene such as the beta= galactosidase gene of E. coli, juxtaposed to regulatory sequences including a transcriptional promoter and possibly a polyadenylation signal, may be included in the vector but is not essential for expression of a convected gene. A typical vector for such purposes pAC373, is shown in Figure 8.
C. Genetically Engineered Baculovirus Expresses BPI Protein Creation of recombinant baculovirus: A chimeric baculovirus is created by homologous recombination between the expression plasmid containing the BPI protein target gene (or truncations thereof) and wild type baculovirus DNA. Plasmid and wild type baculovirus DNA are co-precipitated by the calcium phosphate technique and added to uninfected Spodoptera frugiperda (Sf9) insect cells. Four to seven days following transfection, cells will exhibit a cytopathic morphology and contain the nuclear occlusion bodies typically produced by viral infection. The cell-free culture media containing both wild type and recombinant virus is harvested.

Identification and isolation of chimeric baculovirus: Clonal isolates of virus can be obtained from this co-transfection stock by plaque purification on Sf9 cell monolayers overlaid with agarose. Candidate plaques for analysis will be identified by a plaque morphology negative for occlusion bodies. If the expression plasmid contains a marker gene such as beta galactosidase, recombinant plaques will be indicated by the blue color produced from a chromogenic substrate such as 5-bromo-4-chloryl-3-indolyl-β-D-galactopyranoside (X-gal) in the agarose plating medium. Picked plaques will be used for inoculation of cells in multiwell dishes. The resulting cell lysates and infected cell supernatants can be evaluated for expression of recombinant BPI Protein, using standard activity or immunological assays. Positive wells may require additional rounds of plaque purification to obtain pure recombinant virus stocks free from wild type contamination.

Batch production of BPI Protein: Sf9 cells are adapted to growth in serum-free, low protein medium such as ExCell (J.R. Scientific). Cells are collected from suspension culture by gentle centrifugation and resuspended in fresh medium containing the viral inoculum at a concentration of ten million cells per ml, using a multiplicity of infection of one virus plaque forming unit per cell. After a period of two hours, the culture is diluted five fold with fresh medium and incubated two to three days. At the end of that time, the cells are pelleted by centrifugation and the conditioned medium harvested. BPI protein is purified from the cell-free supernatant by standard means.

Characterization of insect cell derived PN-I: BPI protein produced in insect cells using a baculovirus expression system is a glycosylated protein of approximate molecular weight of 55kd. The N-terminal amino acid sequence is identical to that of mature mammalian cell BPI Protein, indicating correct processing of the signal sequence. The specific activity of endotoxin binding is indistinguishable from native BPI Protein.

Construction of pT7BPI Protein Plasmids: (see Figure 2) oligonucleotides were prepared on an Applied Biosystems 380B DNA Synthesizer for use in polymerase chain reactions (PCR). The oligonucleotides created Nde I and BamHI restriction sites at the 5' and 3' ends, respectively, of the BPI protein DNA. In addition, another oligonucleotide containing a BamHI restriction site was used to create the truncated proline-212 version of the BPI protein DNA.

Following the PCR reactions, fragments were purified and digested with Nde I and BamHI. The plasmid, pGEMEX-1, (available from Promega) was selected as the vector for the constructions. pGEMEX-1 contains a T7 promoter which can be used for the expression of downstream sequences when placed into the proper host. The vector was cleaved with BamHI and, following a purification, partially digested with Nde I to generate a vector with a single Nde I site and a single BamHI site. The fragments were ligated and transformed into the E. coli strain JM101 using the Hanahan transformation protocol. The transformed bacteria were plated on LB plates containing carbamicillin and incubated overnight at 37°c. Resistant colonies were selected and analyzed by preparing mini-plasmid preparations and digesting with the appropriate restriction enzymes. Digests were analyzed on both 1% agarose gels and 5% PAGE.

The expression host, E. coli strain JM109(DE3), was transformed using 1 µ1 of the mini-plasmid preparation and the Hanahan transformation protocol. JM109(DE3) contains a chromosomal copy of the gene for T7 RNA polymerase which can be induced with IPTG. The transformed bacteria were plated on LB plates containing carbamicillin and incubated overnight at 37°C.

## Claims

1. A method of detecting lipopolysaccharide in a sample which comprises contacting the sample with composition comprising a Bactericidal/Permeability Increasing Protein and a lipid carrier, wherein the Bactericidal/Permeability Increasing Protein is solubilized in the lipid carrier, under conditions such that a lipopolysaccharide-Bactericidal/Permeability Increasing Protein complex is formed, and detecting the lipopolysaccharide-Bactericidal/Permeability Increasing Protein complex, thereby detecting the lipopolysaccharide in the sample.

2. A method of quantitatively determining the amount of lipopolysaccharide in a sample which comprises contacting the sample with a composition comprising a Bactericidal/Permeability Increasing Protein and a lipid carrier, wherein the Bactericidal/Permeability Increasing Protein is solubilized in the lipid carrier, under conditions such that a lipopolysaccharide-Bactericidal/Permeability Increasing Protein complex is formed, and quantitatively determining the amount of the lipopolysaccharide-Bactericidal/Permeability Increasing Protein complex, thereby quantitatively determining the amount of lipopolysaccharide in the sample.

3. The method of anyone of claims 1 or 2, wherein the lipid carrier comprises a liposome.

4. The method of anyone of claims 1 or 2, wherein the lipid carrier comprises a non-ionic detergent.

## Patentansprüche

1. Verfahren zum Nachweis eines Lipopolysaccharids in einer Probe durch Kontaktieren der Probe mit einer Zusammensetzung, umfassend ein bakterizides/permeabilitätssteigerndes Protein und einen Lipidträger, wobei das bakterizide/permeabilitätssteigernde Protein in dem Lipidträger solubilisiert ist, unter Bedingungen, unter denen ein Komplex aus Lipopolysaccharid und bakterizidempermeabilitätssteigerndem Protein entsteht, und Bestimmen des Komplexes aus dem Lipopolysaccharid und dem bakteriziden/permeabilitätssteigernden Protein zum Nachweis des Lipopolysaccharids in der Probe.

2. Verfahren zur quantitativen Bestimmung der Menge eines Lipopolysaccharids in einer Probe durch Kontaktieren der Probe mit einer Zusammensetzung, umfassend ein bakterizides/permeabilitätssteigerndes Protein und einen Lipidträger, wobei das bakterizide/permeabilitätssteigernde Protein in dem Lipidtrager solubilisiert ist, unter Bedingungen, unter denen ein Komplex aus dem Lipopolysaccharid und dem bakteriziden/permeabilitätssteigernden Protein entsteht, und quantitatives Bestimmen der Menge an dem Komplex aus dem Lipopolysaccharid und dem bakteriziden/permeabilitätssteigernden Protein zur quantitativen Ermittlung der Menge an dem Lipopolysaccharid in der Probe.

3. Verfahren nach einem der Ansprüche 1 oder 2, wobei der Lipidträger ein Liposom umfaßt.

4. Verfahren nach einem der Ansprüche 1 oder 2, wobei der Lipidträger ein nichtionisches Detergens umfaßt.

## Revendications

1. Procédé pour détecter un lipopolysaccharide dans un échantillon, qui comprend la mise en contact de cet échantillon avec une composition comprenant une Protéine Bactéricide / Accroissant la Perméabilité et un porteur lipidique, dans laquelle la Protéine Bactéricide / Accroissant la Perméabilité est dissoute dans le porteur lipidique, dans des conditions telles qu'un complexe lipopolysaccharide-Protéine Bactéricide / Accroissant la Perméabilité se forme ; et la détection du complexe lipopolysaccharide-Protéine Bactéricide / Accroissant la Perméabilité, détectant ainsi le lipopolysaccharide dans l'échantillon.

2. Procédé pour déterminer quantitativement la teneur en lipopolysaccharide d'un échantillon, qui comprend la mise en contact de cet échantillon avec une composition comprenant une Protéine Bactéricide / Accroissant la Perméabilité et un porteur lipidique, dans laquelle la Protéine Bactéricide / Accroissant la Perméabilité est dissoute dans le porteur lipidique, dans des conditions telles qu'un complexe lipopolysaccharide-Protéine Bactéricide / Accroissant la Perméabilité se forme ; et la détermination quantitative de la teneur en complexe lipo-polysaccharide-Protéine Bactéricide / Accroissant la Perméabilité, déterminant ainsi quantitativement la teneur en lipopolysaccharide de l'échantillon.

3. Procédé selon l'une quelconque des revendications 1 ou 2, dans lequel le porteur lipidique comprend un liposome.

4. Procédé selon l'une quelconque des revendications 1 ou 2, dans lequel le porteur lipidique comprend un détergent non ionique.
